# EUROPÄISCHE PATENTSCHRIFT

(11) **EP 0 268 992 B1**
(45) Veröffentlichungstag und Bekanntmachung des Hinweises auf die Patenterteilung: **02.02.1994**
(21) Anmeldenummer: 87117033.8
(22) Anmeldetag: 19.11.1987
(51) Int. Cl.: A61K 7/08

(54) **Fliessfähiges Perlglanzkonzentrat**
Flowing nacreous concentrats
Concentrés nacrés fluidisables

(30) Priorität: 28.11.1986 DE 3640755
(43) Veröffentlichungstag der Anmeldung: 01.06.1988
(73) Patentinhaber: Henkel Kommanditgesellschaft auf Aktien, 40191 Düsseldorf (DE)
(72) Erfinder: Höffkes, Horst, Dr., D-4000 Düsseldorf (DE); Kaczich, Anke, D-4000 Düsseldorf (DE)

(56) Entgegenhaltungen:
- EP-A- 0 158 174
- EP-A- 0 164 058
- EP-A- 0 195 251
- EP-A- 0 205 922
- DE-A- 1 669 152

## Beschreibung

Gegenstand der Erfindung ist ein Perlglanzkonzentrat in Form einer fließfähigen wäßrigen Dispersion.

Wäßrigen Zubereitungen von Tensiden und kosmetischen Präparaten kann man durch Einarbeitung von Substanzen, die nach dem Abkühlen in Form feiner, perlmuttartig aussehender Kristalle ausfallen und in den Zubereitungen dispergiert bleiben, ein perglänzendes, ästhetisch ansprechendes Aussehen verleihen. Als perlglanzbildende Stoffe eignen sich z. B. die Mono- und Diester aus Ethylenglycol, Propylenglycol und oligomeren Alkylenglycolen dieser Art oder Glycerin mit C₁₆-C₂₂-Fettsäuren sowie Monoalkanolamide von C₁₂-C₂₂-Fettsäuren mit Alkanolaminen mit 2 oder 3 C-Atomen.

Es ist auch bekannt, die genannten Perlglanzbildner in Wasser oder in wäßrigen Tensidlösungen stabil zu dispergieren und die auf diese Weise erhaltenen konzentrierten Perlglanzdispersionen den perlglänzend auszustattenden Zubereitungen ohne Erwärmung zuzusetzen, so daß sich das für die Einarbeitung sonst erforderliche Erwärmen und Abkühlen zur Bildung der Perlglanzkristalle erübrigt.

Perlglanzkonzentrate auf Basis der genannten Perlglanzbildner sind z. B. aus DE-A-16 69 152 und aus JP-56/71021 (Chem. Abstr. 95/156360) bekannt. Die aus JP-56/71021 bekannten Perlglanzkonzentrate haben den Nachteil, daß sie nicht fließfähig sind und durch entsprechende Verdünnung mit Wasser keine stabilen fließfähigen Dispersionen ergeben. Dadurch wird die Handhabung und technische Verarbeitung der Konzentrate erheblich erschwert. Die aus DE-A-16 69 152 bekannten Perlglanzkonzentrate enthalten zur Stabilisierung der Disperion im flüssigen Zustand anionische Tenside. Diese haben den Nachteil, daß sie in Rezepturen mit Rezepturbestandteilen entgegengesetzter lonogenität unverträglich und zu Störungen der Dispersionsstabilität oder in Rezepturen mit kationischen Avivagewirkstoffen zu einer Verringerung der Avivagewirkung führen.

Aus EP-A-195 251 sind Haarspülmittel mit Perlglanzeffekt bekannt, zu deren Herstellung ein Perlglanzkonzentrat eingesetzt wurde, das bestimmte nichtionogene Ethylenoxid-addukte mit einem HLB-Wert von 4 bis 12 zur Stabilisierung enthielt. Aus EP-A-158 174 sind Perlglanzdispersionen, die zur Stabilisierung ein nichtionisches Anlagerungsprodukt von 4 Mol Ethylenoxid und 4 Mol Propylenoxid an einen C_{10/12}-Fettalkohol enthalten. Aus EP-A-164 058 sind Perlglanzdispersionen bekannt, die mit einem Sulfosuccinat- oder Aminoxid-Tensid stabilisiert sind.

Es hat sich gezeigt, daß viele nichtionogene und zwitterionische Emulgatoren entweder zu Perlglanzdispersionen von geringer Stabilität und Brillanz oder zu Perlglanzdispersionen führen, die in kationischen Haarpflegemittelrezepturen die Wirkung der kationischen Avivagemittel verringern. Es bestand daher die Aufgabe. solche Emulgatoren zu finden, welche die Herstellung fließfähiger Perlglanzdispersionen von hoher Brillanz und hoher Stabilität und mit guter Verträglichkeit mit kationischen Avivagewirkstoffen ermöglichen.

Gegenstand der Erfindung ist ein Perlglanzkonzentrat in Form einer fließfähigen Dispersion mit 5 bis 15 Gew.-% eines oder mehrerer Ester der Formel I

R¹ - (OCₙH₂ₙ)ₓ - OR² (I)

in der R¹ eine lineare Fettacylgruppe mit 16 bis 22 C-Atomen, R² Wasserstoff oder eine Gruppe R¹, n = 2 oder 3 und x eine Zahl von 1 bis 4 ist und mit 1 bis 6 Gew.-% eines oder mehrerer Monoethanolamide von Fettsäuren mit 12 bis 22 C-Atomen, dadurch gekennzeichnet, daß als Emulgatoren 2 bis 8 Gew.-% einer oder mehrerer Verbindungen der allgemeinen Formel II enthalten sind, wobei

H - (C₆H₁₀O₅)_{y} - OR³ (II)

ein Alkyl(oligo)-glucosid ist, worin y der mittlere Oligomerisationsgrad = 1 bis 5 und R³ eine Alkylgruppe mit 6 bis 12 C-Atomen,
ist.

Daneben enthalten die erfindungsgemäßen Perlglanzkonzentrate im wesentlichen Wasser in einer Menge von ca. 70 bis 90 Gew.-%. Die mit den vorgenannten Emulgatoren der Formel II herstellbaren Perlglanzkonzentrate sind bei Raumtemperatur von ca. 10 bis 20 °C fließfähig und weisen einen bis mindestens 50 °C stabilen Perlglanz auf, der auch in den damit ausgestatteten Zubereitungen bei schwankenden Temperaturen erhalten bleibt. Die Perlglanzkristalle weisen eine hohe Brillanz auf und bilden sich auch nach dem Aufschmelzen bzw. Lösen durch Erwärmung über den Schmelzpunkt beim Abkühlen in derselben brillanten, einheitlichen Kristallform wieder aus.

Als Ester der Formel (I) R¹(OCₙH₂ₙ)ₓOR² können z. B. die Mono- und bester des Ethylenglycols und Propylenglycols mit höheren Fettsäuren, z. B. mit Palmitinsäure, Stearinsäure oder Behensäure oder die Diester des Diethylenglycols oder des Triethylenglycols mit solchen Fettsäuren eingesetzt werden. Geeignet sind auch Mischungen von Mono- und Diestern der genannten Glycole mit Fettsäuregemischen, z. B. mit gehärteter Talgfettsäure oder mit der gesättigten C₁₆-C₁₈-Fettsäurefraktion der Talgfettsäure. Bevorzugt geeignet ist der Ethylenglycolmono- und/oder Diester der Palmitin- und/oder Stearinsäure.

Als Monoethanolamide von Fettsäuren mit 12 - 18 C-Atomen können z. B. Laurinsäuremonoethanolamid, Myristinsäuremonoethanolamid, Palmitin-/Stearinsäure-Monoethanolamid und bevorzugt das Monoethanolamid der C₁₂-C₁₈-Fraktion der Kokosfettsäure verwendet werden.

Als Alkylglucoside der Formel II eignen sich besonders die Produkte, in welchen R³ eine Alkylgruppe mit 8 bis 10 C-Atomen und der Oligomerisationsgrad y = 1,2 bis 2 ist. Solche Produkte sind, z. B. unter dem Warenzeichen Triton BG 10 und Triton CG 110 (Rohm und Haas) im Handel erhältlich.

Die erfindungsgemäßen Perlglanzkonzentrate weisen einen besonders seidig bis metallisch-glänzenden Perlglanz auf, wenn als perlglanzbildender Ester der Formel I eine Kombination aus
- 5 bis 8 Gew.-% eines Ethylenglykolmono- und distearat, bevorzugt im Gewichtsverhältnis 1 : 2 bis 1 : 5 und
- 2 bis 5 Gew.-% Triethylenglykoldistearat
enthalten ist.

Anstelle des Ethylenglycolmono- und -distearats und des Triethylenglycoldistearats können auch die entsprechenden Ester von Palmitinsäure-Stearinsäure-Gemischen eingesetzt werden, wobei wenigstens 50 Gew.-% Stearinsäure im Gemisch vorliegen soll.

Besonders stabile Perlglanzkonzentrate werden erhalten, wenn
6 bis 10 Gew.-% des perlglanzbildenden Esters der Formel I,
3 bis 5 Gew.-% eines C₁₂-C₁₈-Kokosfettsäuremonoethanolsamids und
4 bis 6 Gew.-% eines Emulgators der Formel II
enthalten ist.

Neben den genannten obligatorischen Komponenten enthalten die erfindungsgemäßen Perlglanzkonzentrate im wesentlichen Wasser. In untergeordneten Mengen sind Konservierungsmittel, z. B. Formaldehyd, Na-Benzoat, Sorbinsäure, p-Hydroxybenzoesäureester, 5-Brom-5-nitro-1,3-dioxan oder andere für wäßrige Zubereitungen geeignete Konservierungsmittel enthalten. Weiterhin können in untergeordneten Mengen Puffersubstanzen zur Einstellung des pH-Wertes auf Werte zwischen 6 und 8, z. B. Citronensäure und/oder Natriumcitrat enthalten sein.

Die Herstellung der erfindungsgemäßen Perlglanzkonzentrate erfolgt in der Weise, daß die perlglanzbildenden Fettstoffe und Emulgatoren zunächst gemeinsam über ihren Schmelzpunkt, bevorzugt auf eine Temperatur von 75 - 100 °C erwärmt und vermischt werden. Zu dieser Schmelze wird dann das auf ebenfalls 75 - 100 °C erwärmte Wasser unter Rühren zugegeben. Das Wasser kann bereits Konservierungsmittel und Puffersubstanzen enthalten. Die entstehende Emulsion wird dann innerhalb von 5 - 20 Minuten auf ca. +50 °C unter Rühren gekühlt und bei dieser Temperatur mit einem Homogenisator oder Dispergieraggregat, welches hohe Scherkräfte entwickelt, für kurze Zeit, z. B. von 1 - 3 Minuten, homogenisiert. Hierfür eignen sich statische und dynamische Mischaggregate, z. B. Spalthomogenisatoren oder Dispergiergeräte, die nach dem Stator-Rotor-Prinzip arbeiten. Nach dieser kurzen und intensiven Homogenisierung wird die gebildete Dispersion unter langsamem Rühren weiter auf Raumtemperatur abgekühlt.

Ein besonders elegantes Verfahren zur Herstellung der erfindungsgemäßen Perlglanzkonzentrate besteht darin, daß man
- die perlglanzbildenden Fettstoffe, Emulgatoren und eine erste Teilmenge des Wassers einzeln oder gemeinsam erhitzt und unter Bildung einer Emulsion innig vermischt, deren Temperatur um 1 bis 30 °C über der Schmelz- bzw. Kristallisationstemperatur der perlglanzbildenden Fettstoffe liegt und danach
- die zweite Teilmenge des Wassers zumischt, wobei deren Temperatur so bemessen ist, daß die dabei entstehende Dispersion eine Temperatur annimmt, die um 3 bis 15 °C unterhalb der Kristallisationstemperatur der perlglanzbildenden Fettstoffe liegt.

Dieses Verfahren eignet sich besonders gut für die kontinuierliche Herstellung.

Die erfindungsgemäßen Perlglanzkonzentrate eignen sich zur Erzeugung von Perlglanz in wäßrigen Tensidzubereitungen beliebiger lonogenität und in wäßrigen kosmetischen Zubereitungen gleichgültig ob diese kationische oder anionische Tenside oder Polymere enthalten. Zur Erzeugung von Perlglanz werden 1 - 10 Gew.-% der erfindungsgemäßen Perlglanzkonzentrate in der wäßrigen Zubereitung verteilt. Die Verteilung der Perlglanzkonzentrate ist unter leichtem Rühren ohne Erwärmung, also bei Raumtemperaturen von 10 - 30 °C, möglich.

Die folgenden Beispiele sollen den Erfindungsgegenstand näher erläutern.

### Beispiele

1. Es wurden Perlglanzkonzentrate der Zusammensetzung

| | |
|---|---|
| Ethylenglycolstearat (CTFA-Bezeichnung: Glycol Distearate) | 8,0 kg |
| Kokosfettsäure(C₁₂-C₁₈)-monoethanolamid | 4,0 kg |
| Emulgator | 5,0 kg |
| Wasser | 83,0 kg |

hergestellt, wobei als Emulgator nacheinander die folgenden Verbindungen eingesetzt wurden
1.1 Alkyl(C₈-C₁₀)-oligo(1,8)-glucosid
(Triton^{(R)}BG 10, Rohm & Haas)
1.2 Alkyl(C₈-C₁₀)oligo(1,3)-glucosid
(Triton^{(R)}CG 110, Rohm & Haas)

Die Herstellung erfolgte auf folgende Weise:
Die Mischung aus Ethylenglykolstearat, Kokosfettsäure-monoethanolamid und dem Emulgator wurde auf 75 °C erhitzt. Dann wurden 8 kg Wasser auf 75 °C erwärmt und zugegeben und durch Homogenisierung mit einer Turbine eine Emulsion gebildet. Dann wurde die restliche Wassermenge von 75 kg auf 25 °C erwärmt und unter Rühren zu der Emulsion gegeben. Es bildete sich eine Dispersion, deren Temperatur 47 °C betrug und die nach wenigen Minuten ein perlglänzendes Aussehen annahm. Die Dispersion wurde dann in ein Vorratsfaß gefüllt und war für die weitere Verwendung geeignet.

### Anwendungsbeispiele

Die Perlglanzkonzentrate 1.1 und 1.2 wurden in folgende Rezepturen eingesetzt:

### 2.1 Perlglänzende Haarnachspülmittel

Emulsionsförmiges Haarnachspülmittel aus

| | |
|---|---|
| 3 Gew.-% Cetyl-/Stearylalkohol | |
| 0,5 Gew.-% Cetyltrimethylammoniumchlorid | 8 kg |
| 96,5 Gew.-% Wasser | |
| Perlglanzkonzentrat 1.1 und 1.2 | 2 kg |

Es wurden Haarnachspülmittel mit ansprechendem Perlglanz und guter Avivagewirkung erhalten.

### 2.2 Perlglänzendes Haarshampoo

| | |
|---|---|
| Fettalkohol(C₁₂/C₁₄)-poly(2 EO)glykolethersulfat, Na-Salz (28 Gew.-%ige wäßrige Lösung) | 40 kg |
| N-Kokos(C₁₂₋₁₈)acylamidopropyl-dimethylglycin (30 Gew.-%ige wäßrige Lösung) | 10 kg |
| Perlglanzkonzentrat 1.1 und 1.2 | 5 kg |
| Wasser | 45 kg |

Es wurden perlglänzende Haarshampoos erhalten.

## Patentansprüche

1. Perlglanzkonzentrat in Form einer fließfähigen Dispersion mit 5 bis 15 Gew.-% eines oder mehrerer Ester der Formel I
R¹ - (OCₙH₂ₙ)ₓ - OR² (I)
in der R¹ eine lineare Fettacylgruppe mit 16 bis 22 C-Atomen, R² Wasserstoff oder eine Gruppe R¹, n = 2 oder 3 und x eine Zahl von 1 bis 4 ist und mit 1 bis 6 Gew.-% eines oder mehrerer Monoethanolamide von Fettsäuren mit 12 bis 22 C-Atomen, dadurch gekennzeichnet, daß als Emulgatoren 2 bis 8 Gew.-% einer oder mehrerer Verbindungen der allgemeinen Formel II enthalten sind, wobei
H - (C₆H₁₀O₅)_{y} - OR³ (II)
ein Alkyl(oligo)-glucosid ist, worin y der mittlere Oligomerisationsgrad = 1 bis 5 und R³ eine Alkylgruppe mit 6 bis 12 C-Atomen ist.

2. Perlglanzkonzentrat nach Anspruch 1, dadurch gekennzeichnet, daß
6 bis 10 Gew.-% eines Perlglanzbildners der Formel (I),
3 bis 5 Gew.-% eines C₁₂-C₁₈-Kokosfettsäuremonoethanolamids und
4 bis 6 Gew.-% eines Emulgators der Formel (II)
enthalten ist.

## Claims

1. A pearlescent concentrate in the form of a free-flowing dispersion containing 5 to 15% by weight of one or more esters corresponding to formula I:
R¹ - (OCₙH₂ₙ)ₓ - OR² (I)
in which R¹ is a linear fatty acyl group containing 16 to 22 carbon atoms, R² is hydrogen or a group R¹, n = 2 or 3 and x is a number of 1 to 4,
and 1 to 6% by weight of one or more monoethanolamides of C₁₂₋₂₂ fatty acids, characterized in that one or more compounds corresponding to general formula II:
H - (C₆H₁₀O₅)_{y} - OR³ (II)
being an alkyl (oligo)glucoside in which y is the average degree of oligomerization and has a value of 1 to 5 and R³ is a C₆₋₁₂ alkyl group,
is/are present as emulsifiers in a quantity of 2 to 8% by weight.

2. A pearlescent concentrate as claimed in claim 1, characterized in that it contains
6 to 10% by weight of a pearlescer corresponding to formula (I),
3 to 5% by weight of a C₁₂₋₁₈ coconut oil fatty acid monoethanolamide and
4 to 6% by weight of an emulsifier corresponding to formula (II).

## Revendications

1. Concentré nacré sous forme d'une dispersion apte à l'écoulement, ayant de 5 à 15 % en poids d'un ou plusieurs esters de formule (I) :
R¹-(OCnH₂ₙ)ₓ-OR² (I)
dans laquelle R¹ est un groupe acyle d'acide gras linéaire ayant de 16 à 22 atomes de Carbone,
R₂ est de l'hydrogène ou un radical R¹,
n est égal à 2 ou 3,
et x est un nombre de 1 à 4,
et de 1 à 6 % en poids d'un ou plusieurs monoéthanolamides d'acides gras ayant de 12 à 22 atomes de Carbone,
caractérisé en ce que comme agent émulsionnant, 2 à 8 % en poids d'un ou de plusieurs composés de formule générale II y sont contenus dans laquelle :
H-(C₆H₁₀O₅)_{y}-OR₃ (II)
est un alcoyl-(oligo) glucoside dans lequel y le degré moyen d'oligomérisation est compris de 1 à 5, et R³ est un radical alcoyle ayant de 6 à 12 atomes de carbone.

2. Concentré nacré selon la revendication 1, caractérisé en ce qu'il contient :
6 à 10 % en poids d'un agent de formation du brillant de nacre de formule I,
3 à 5 % en poids d'un monoéthanolamine d'acide gras de Coco en C₁₂ à C₁₈, et 4 à 6 % en poids d'un agent émulsionnant de formule (II).
